# EUROPEAN PATENT SPECIFICATION

(11) **EP 4 103 152 B1**
(45) Date of publication and mention of the grant of the patent: **23.07.2025**
(21) Application number: 21705451.9
(22) Date of filing: 10.02.2021
(51) Int. Cl.: A61K 9/00, A61K 9/1271, A61K 41/00

(54) **GAS-FILLED MICROVESICLES FOR THERAPEUTIC USE**
GASGEFÜLLTE MIKROVESIKEL ZUR THERAPEUTISCHEN VERWENDUNG
MICROVÉSICULES REMPLIES DE GAZ POUR UTILISATION THÉRAPEUTIQUE

(30) Priority: 11.02.2020 US 202062975005 P
(43) Date of publication of application: 21.12.2022
(73) Proprietor: Bracco Suisse SA, 6814 Cadempino (CH)
(72) Inventor: JEANNOT, Victor, 1228 Plan-les-Ouates (CH); HELBERT, Alexandre, 1228 Plan-les-Ouates (CH)
(74) Representative: Ravizza, Claudio
(86) International application number: PCT/EP2021/053183
(87) International publication number: WO 2021/160659

(56) References cited:
- WO-A1-2016/097130
- WO-A1-2016/102515
- WO-A2-2010/040772
- WO-A2-2014/096165
- SCHNEIDER MICHEL ET AL: "BR38, a New Ultrasound Blood Pool Agent", INVESTIGATIVE RADIOLOGY, LIPPINCOTT WILLIAMS & WILKINS, US, vol. 46, no. 8, 31 July 2011 (2011-07-31), pages 486 - 494, XP009520989, ISSN: 0020-9996, DOI: 10.1097/RLI.0B013E318217B821
- DIMCEVSKI GEORG ET AL: "A human clinical trial using ultrasound and microbubbles to enhance gemcitabine treatment of inoperable pancreatic cancer", JOURNAL OF CONTROLLED RELEASE, ELSEVIER, AMSTERDAM, NL, vol. 243, 12 October 2016 (2016-10-12), pages 172 - 181, XP029862770, ISSN: 0168-3659, DOI: 10.1016/J.JCONREL.2016.10.007
- EMEA: "SonoVue, ANNEX I SUMMARY OF PRODUCT CHARACTERISTICS", 1 January 2011 (2011-01-01), XP055704833, Retrieved from the Internet <URL:https://www.ema.europa.eu/en/documents/product-information/sonovue-epar-product-information_en.pdf> [retrieved on 20200615]

## Description

### Technical field

The present invention relates to the field of gas-filled microvesicles and in particular to the use of such microvesicles for therapeutic treatments.

### Background of the invention

Therapeutic use of ultrasound waves in combination with a suspension of gas-filled microvesicles is an emerging modality which is applied in many therapeutic treatments. According to certain treatments, gas-filled microvesicles are systemically injected into the blood stream. These treatments may be used for instance for increasing vessels permeabilization in combination with ultrasound, e.g. for promoting/increasing delivery of medicaments (Ref. 1: Wang et al.) or genes, for helping disrupting blood clots (Ref. 2: Auboire et al.), opening blood brain barrier (Ref. 3: Huang et al.), immunomodulation (Ref 4: Tu et al.), neuromodulation (Ref.5: Blackmore et al.), radiosensitization (Ref 6: Deng et al.), or also for helping in non-thermal tissue ablation (Ref. 7: Arvanitis et al.).

As the therapeutic treatment may last for few minutes (e.g. from about ten to more than one hour depending on the location and/or the size of the area to be treated), there is a need to provide a sustained administration of the suspension of gas-filled microvesicles to the patient, for the whole time-span of the treatment. Such sustained administration may be achieved either with a continuous infusion of the suspension or by injecting two or more subsequent boluses of a certain volume of the suspension.

In general, a bolus administration is a relatively simple procedure which comprises administering to a subject a certain volume of a suspension of gas-filled microvesicles within few seconds, e.g. by using a syringe and a catheter placed in a peripheral vein. Infusion is a more complex procedure which requires the use of a dedicated device, e.g. an injector or a pump to inject the suspension, a longer preparation time prior to the treatment and the presence of skilled operator during a long period (before/after treatment). Furthermore, infusion for long time periods shall take into account the decantation phenomenon of gas microvesicles in the syringe, with the need of a dedicated device (e.g. rotating syringe) or dedicated procedures (e.g. manual agitation of saline bag containing the microvesicles suspension). All in all, this represents a substantial additional cost. As observed by the Applicant conventional preparations of gas-filled microvesicles, when administered as bolus in therapeutic applications, need to be administered at relatively frequent intervals to allow the presence of a sufficient amount of microvesicles in the anatomical region under treatment. For instance, Thomas et al. describes the use of SonoVue^{®} (two boluses administered within 45 s) to enhance delivery of liposomes (Ref 8: Thomas et al., 2018). Furthermore, Ref. 9 (Dimcevski et al. 2016) discloses the use of SonoVue^{®} in the ultrasound mediated delivery therapeutic treatment with an anticancer medicament (gemcitabine); for this purpose, a bolus of SonoVue^{®} was injected every 3.5 minutes.

Ref. 10 (Schneider et al.) discloses a suspension of phospholipid-stabilized microvesicles obtained by reconstitution of a freeze-dried composition comprising PEG4000, DSPC, DPPE-MPEG5000 and palmitic acid, filled with a mixture of nitrogen and perfluorobutane, for use as an imaging ultrasound contrast agent.

Applicant has now found that by using a suspension of gas filled microvesicles comprising the above components it is possible to increase the interval between one bolus and the subsequent one, while maintaining substantially the same therapeutic effect as other known formulations administered at shorter intervals. Advantageously, longer time intervals between administration of subsequent boluses allows a substantial reduction of the total amount of microvesicles, which need to be administered for a treatment requiring a specific time span.

### Summary of the invention

An aspect of the invention relates to a suspension of gas-filled microvesicles comprising a fatty acid di-ester of phosphatidylcholine (PC), a pegylated phosphatidylethanolamine (PE-PEG) and a fatty acid for use in a method for therapeutic treatment of a subject, wherein said suspension is administered as at least two subsequent boluses at an interval of at least four minutes and up to fifteen minutes between each other.

Preferably said composition comprises DSPC, DSPE-MPEG5000 and palmitic acid. Said microbubbles are filled with a physiologically acceptable gas substantially insoluble in water, preferably a perfluorinated hydrocarbon, more preferably perfluorobutane.

Preferably said gas-filled microvesicles comprise the above components in a relative molar ratio (PC/PE-PEG/Palmitic acid) of 60-90/2-10/5-30, preferably 70-80/3-9/10-25 and more preferably of about 72-78/5-8/15-20.

### Figures

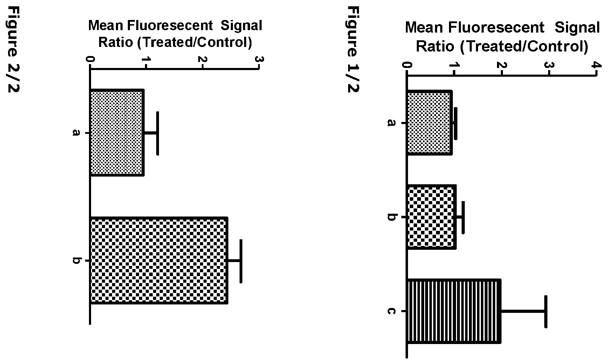

Figure 1 shows the results of the extravasation experiment in example 2a (high frequency, high acoustic pressure), column a representing the efficacy of a bolus treatment with Sonovue^{®}, column b the efficacy of a bolus treatment with Definity^{®} and column c the efficacy of the bolus treatment with a formulation according to the invention.
Figure 2 shows the results of the extravasation experiment in example 2b (low frequency, low acoustic pressure), column a representing the efficacy of a bolus treatment with Definity^{®} and column b the efficacy of the bolus treatment with a formulation according to the invention.

### Detailed description of the invention

As mentioned above, the present invention is based on the finding that a specific formulation of gas-filled microvesicles can be administered as subsequent boluses with advantageously longer time intervals with respect to known formulations.

As observed by the Applicant, while prior art formulations may satisfactorily work when injected as boluses at relatively short intervals, when such interval is increased (e.g. four minutes or longer) the effectiveness of the treatment may be impaired.

The Applicant has now found that a microvesicle formulation comprising a fatty acid di-ester of phosphatidylcholine, a pegylated phosphatidylethanolamine and a fatty acid (preferably DSPC, DPPE-MPEG5000 and palmitic acid), filled with a gas comprising a fluorinated gas (preferably a mixture of nitrogen and perfluorobutane), is capable of being administered as subsequent boluses at an interval of at least four minutes and up to fifteen minutes between each other, without substantially impairing the effectiveness of the therapeutic treatment. Such interval is preferably of at least five minutes and up to fifteen minutes, preferably up to ten minutes.

Advantageously, use of longer administration boluses intervals allows substantially reducing the total amount of injected material as well as reducing the number of required administrations while maintaining the effectiveness of the therapeutic treatment.

### Formulation

Examples of fatty acids di-esters of phosphatidylcholine comprise dilauroyl-phosphatidyl-choline (DLPC), dimyristoyl-phosphatidylcholine (DMPC), dipalmitoyl-phosphatidyl-choline (DPPC), diarachidoyl-phosphatidylcholine (DAPC), distearoyl-phosphatidyl-choline (DSPC), dioleoyl-phosphatidylcholine (DOPC), 1,2 Distearoyl-sn-glycero-3-ethylphosphocholine (Ethyl-DSPC), dipentadecanoyl-phosphatidylcholine (DPDPC), 1-myristoyl-2-palmitoyl-phosphatidylcholine (MPPC), 1-palmitoyl-2-myristoyl-phosphatidylcholine (PMPC), 1-palmitoyl-2-stearoyl-phosphatidylcholine (PSPC), 1-stearoyl-2-palmitoyl-phosphatidylcholine (SPPC), 1-palmitoyl-2-oleyl-phosphatidylcholine (POPC), 1-oleyl-2-palmitoyl-phosphatidylcholine (OPPC). Preferred are DPPC, DAPC, DSPC, and DOPC, particularly preferred being DSPC.

Examples of pegylated phosphatidylethanolamines include phosphatidylethanolamines where the hydrophilic ethanolamine moiety is linked to a polyethyleneglycol (PEG) molecule of variable molecular weight (e.g. 1000 to 5000 g/mol), such as pegylated dipalmitoylphosphatidylethanolamine (DPPE-PEG) or distearoyl phosphatidyl-ethanolamine (DSPE-PEG). Preferably the PEG is a methoxy-terminated PEG, also indicated as MPEG. Where not otherwise indicated, the expression PE-PEG as used herein comprises within its meaning also the PE-MPEG compound. Preferred are PE-PEGs (or PE-MPEGs) with a molecular weight of about 5000 g/mol, such as DPPE-MPEG5000 or DSPE-MPEG5000.

Examples of fatty acids include (C₁₀-C₂₂)-alkyl carboxylic acids, such as lauric acid, myristic acid, palmitic acid, stearic acid, arachidic acid, behenic acid, oleic acid, linoleic acid and linolenic acid; preferably saturated fatty acids such as myristic acid, palmitic acid, stearic acid and arachidic acid; more preferably palmitic acid.

In an optimized formulation, the above components are preferably in a relative molar ratio (PC/PE-PEG/Palmitic acid) of 60-90/2-10/5-30 (i.e. from 60 to 90% molar amount of PC, e.g. DSPC; from 2 to 10 % molar amount of PE-PEG, e.g. DPPE-PEG5000; and from 5 to 30% molar amount of a fatty acid, e.g. palmitic acid; the total amount of components being 100%), more preferably 70-80/3-9/10-25 and even more preferably of about 72-78/5-8/15-20.

Particularly preferred is a formulation comprising DSPC, DPPE-PEG5000 and palmitic acid in the above relative molar ratios.

### Preparation of microvesicles

The microvesicles according to the invention can be manufactured according to any known method in the art. Typically, the manufacturing method involves the preparation of a dried powdered material comprising the composition of the invention, preferably by lyophilization (freeze drying) of an aqueous or organic suspension comprising said composition. The microvesicles can then be obtained by reconstitution of the lyophilized preparation in an aqueous carrier, upon gentle agitation in the presence of a gas.

Preferably, as disclosed for instance in International patent application WO2004/069284, a composition comprising the mixture of phospholipids and fatty acids can be dispersed in an emulsion of water with a water immiscible organic solvent (e.g. branched or linear alkanes, alkenes, cyclo-alkanes, aromatic hydrocarbons, alkyl ethers, ketones, halogenated hydrocarbons, perfluorinated hydrocarbons or mixtures thereof) under agitation, preferably in admixture with a freeze-drying additive (such as carbohydrates, sugar alcohols, polyglycols, polyoxyalkylene glycols and mixtures thereof; preferably polyethyleneglycol is used as freeze-drying agent, in particular PEG4000). The emulsion can be obtained by submitting the aqueous medium and the solvent in the presence of the phospholipids and fatty acid to any appropriate emulsion-generating technique known in the art, such as, for instance, sonication, shaking, high pressure homogenization, micromixing, membrane emulsification, flow focusing emulsification, high speed stirring or high shear mixing. Preferably, an organic solution containing a phospholipid and a fatty acid is first prepared; separately, a pegylated phospholipid is dissolved in an aqueous solution containing a freeze-drying agent; the organic and aqueous phases are then admixed and emulsified as described above. The so obtained microemulsion may optionally be diluted with a solution containing a freeze-drying agent. The microemulsion, which contains microdroplets of solvent surrounded by a stabilizing layer comprising phospholipids and fatty acids, is then lyophilized according to conventional techniques to obtain a lyophilized material.

The freeze-dried product is generally in the form of a powder or a cake and can be stored (typically in a sealed vial) in contact with the desired gas. The freeze-drying additive (e.g. PEG4000) represents typically the largest amount of the composition, e.g. from about 500 to about 1000 times by weight the amount of the components of the microvesicles (i.e. PC/PE-PEG/Palmitic acid). The product is readily reconstituted in a suitable physiologically acceptable aqueous liquid carrier, such as saline, by gentle shaking.

### Gas

Any biocompatible gas, gas precursor or mixture thereof may be employed to form the microvesicles of the invention (here also identified as "microvesicle-forming gas").

The gas may comprise, for example, air; nitrogen; oxygen; carbon dioxide; hydrogen; nitrous oxide; a noble or inert gas such as helium, argon, xenon or krypton; a low molecular weight hydrocarbon (e.g. containing up to 7 carbon atoms), for example an alkane such as methane, ethane, propane, butane, isobutane, pentane or isopentane, a cycloalkane such as cyclobutane or cyclopentane, an alkene such as propene, butene or isobutene, or an alkyne such as acetylene; an ether; a ketone; an ester; halogenated gases, preferably fluorinated gases, such as or halogenated, fluorinated or perfluorinated low molecular weight hydrocarbons (e.g. containing up to 7 carbon atoms); or a mixture of any of the foregoing. Where a halogenated hydrocarbon is used, preferably at least some, more preferably all, of the halogen atoms in said compound are fluorine atoms.

Fluorinated gases are preferred, in particular perfluorinated gases. Fluorinated gases include materials which contain at least one fluorine atom such as, for instance fluorinated hydrocarbons (organic compounds containing one or more carbon atoms and fluorine); sulfur hexafluoride; fluorinated, preferably perfluorinated, ketones such as perfluoroacetone; and fluorinated, preferably perfluorinated, ethers such as perfluorodiethyl ether. Preferred compounds are perfluorinated gases, such as SF₆ or perfluorocarbons (perfluorinated hydrocarbons), i.e. hydrocarbons where all the hydrogen atoms are replaced by fluorine atoms, which are known to form particularly stable microbubble suspensions, as disclosed, for instance, in EP 0554 213, which is herein incorporated by reference.

The term perfluorocarbon includes saturated, unsaturated, and cyclic perfluorocarbons. Examples of biocompatible, physiologically acceptable perfluorocarbons are: perfluoroalkanes, such as perfluoromethane, perfluoroethane, perfluoropropanes, perfluorobutanes (e.g. perfluoro-n-butane, optionally in admixture with other isomers such as perfluoro-isobutane), perfluoropentanes, perfluorohexanes or perfluoroheptanes; perfluoroalkenes, such as perfluoropropene, perfluorobutenes (e.g. perfluorobut-2ene) or perfluorobutadiene; perfluoroalkynes (e.g. perfluorobut-2-yne); and perfluorocycloalkanes (e.g. perfluorocyclobutane, perfluoromethylcyclobutane, perfluorodimethylcyclobutanes, perfluorotrimethylcyclobutanes, perfluorocyclopentane, perfluoromethylcyclopentane, perfluorodimethylcyclopentanes, perfluorocyclohexane, perfluoromethylcyclohexane and perfluorocycloheptane). Preferred saturated perfluorocarbons include, for example, CF₄, C₂F₆, C₃F₈, C₄F₈, C₄F₁₀ and C₅F₁₂, preferably C₄F₁₀.

It may also be advantageous to use a mixture of any of the above gases in any ratio. For instance, the mixture may comprise a conventional gas, such as nitrogen, air or carbon dioxide and a gas forming a stable microbubble suspension, such as sulfur hexafluoride or a perfluorocarbon as indicated above. Examples of suitable gas mixtures can be found, for instance, in WO 94/09829, which is herein incorporated by reference. The following combinations are particularly preferred: a mixture of gases (A) and (B) in which the gas (B) is a fluorinated gas, selected among those previously illustrated, including mixtures thereof, and (A) is selected from air, oxygen, nitrogen, carbon dioxide or mixtures thereof. The amount of gas (B) can represent from about 0.5% to about 95% v/v of the total mixture, preferably from about 5% to 80%.

Particularly preferred gases are SF₆, C₃F₈, C₄F₁₀ or mixtures thereof, optionally in admixture with air, oxygen, nitrogen, carbon dioxide or mixtures thereof. Particularly preferred is C₄F₁₀ and even more preferred is a mixture of nitrogen with C₄F₁₀, preferably in a 35/65 v/v ratio.

### Pharmaceutical kit and administration

Microvesicles suspensions according to the invention can be stored as such or preferably in form of freeze-dried precursor which can be reconstituted with an aqueous carrier. According to the invention, the precursor of the microvesicles suspension is thus preferably stored in dried powdered form and as such can advantageously be packaged in a two components diagnostic and/or therapeutic kit, preferably for administration by injection. The kit preferably comprises a first container, containing the lyophilized precursor composition in contact with a selected microvesicle-forming gas and a second container, containing a physiologically acceptable aqueous carrier for reconstituting the suspension of microvesicles, in particular saline. Said two components kit can include two separate containers or a dual-chamber container. In the former case the container is preferably a conventional septum-sealed vial, wherein the vial containing the lyophilized residue is sealed with a septum through which the carrier liquid may be injected using an optionally prefilled syringe. In such a case the syringe used as the container of the second component is also used then for injecting the suspension of microvesicles. In the latter case, the dual-chamber container is preferably a dual-chamber syringe and once the freeze-dried product has been reconstituted and then suitably mixed or gently shaken, the container can be used directly for injecting the suspension of microvesicles.

The reconstituted suspension thus contains the gas-filled microvesicles with a stabilizing layer comprising the above-mentioned components (PC, PE-PEG and fatty acid), preferably in the above indicated relative molar ratios, preferably containing a mixture of nitrogen and perfluorobutane (65/35 v/v). Typically, the reconstituted gas-filled microvesicles have a median volume concentration (Dv50) of from about 2.5 to 4, microns, preferably 3.0 to 3.5, and a mean diameter in number (Dn) of from about 1 to 2 microns, preferably 1.2 to 1.8. Typically, substantially all microvesicles (e.g. at least 98%, preferably 99%, more preferably 100%) have a diameter lower than 8 microns. In general, the amount of gas filled microvesicles between 2 and 8 microns can be from about 5% to about 40% (preferably 10-30%) of the total number of microvesicles in the suspension. Depending on the volume of reconstitution, the concentration of gas-filled microvesicles in the suspension may vary from about 1x10⁸ to about 2x10⁹ microvesicles/mL.

The microvesicles suspension of the present invention can be used in a variety of therapeutic techniques for increasing vessels permeabilization in combination with ultrasound. Such therapeutic treatments include, for instance, promoting/increasing delivery of medicaments (e.g. for enhancing therapeutic efficacy of drugs or genes, for instance in the treatment of tumors or neurological disorders), disruption of blood clots, immunomodulation, neuromodulation, radiosensitization or non-thermal tissue ablation.

According to a preferred embodiment, an effective amount of microvesicles is administered to a patient, typically by injection of a suspension thereof, preferably as subsequent boluses, preferably in combination with a medicament, e.g. an antitumoral drug. The administration of the suspension of microvesicles can be either concurrent or subsequent with respect to the administration of the medicament.

The expression "bolus administration" as used herein refers to a single intravascular injection of certain a volume of a suspension of gas-filled microvesicles e.g. by means of a syringe and a catheter placed in a peripheral vein. The administration of a bolus is generally performed within few seconds (e.g. from 5 to 120 seconds, preferably not more than 60 seconds) "Subsequent" or "repeated" boluses refer to bolus injections which are repeated after a certain period of time, e.g. after at least four minutes and up to fifteen minutes between each other according to the invention.

The total amount of microvesicles for each injected volume of suspension (bolus) can vary from 6x10⁵ to 2x10⁹ microvesicles per kg of patient, preferably from 1x10⁷ to 1x10⁹ microvesicles per kg, even more preferably from 2x10⁷ to 4x10⁸ microvesicles per kg.

The volume of each injected bolus is adapted to the required total amount of microvesicles to be injected (which generally depends on the specific treatment to be performed), taking into account the concentration of microvesicles in the injected suspension and the weight of the patient. As a general rule, for concentrations of microvesicles in the suspension of from about 1x10⁸ to about 2x10⁹ microvesicles/mL, the bolus volume may vary from 0.01 to 120 mL, preferably from 0.2 to 60 mL and even more preferably from 0.4 to 20 mL.

While the present composition can be administered at any time interval as subsequent boluses, the Applicant has observed that the formulation can be used in therapeutic treatments in combination with ultrasound by administering it at longer intervals with respect to known microvesicles formulations. In particular, said time interval between two subsequent boluses can be of at least four minutes, preferably five minutes and up to fifteen minutes, preferably up to ten minutes. On the other side, the skilled person would appreciate that by using the formulation of the invention at shorter intervals between a bolus and the subsequent one, the efficacy of the treatment is nevertheless ameliorated with respect to the one produced by other known formulations of gas-filled microvesicles being injected at the same conditions (i.e. concentration of microvesicles and time interval).

Suitable insonation of the body part to be treated in the presence of the microvesicles will provide the desired enhanced therapeutic effect, e.g. by increasing the therapeutic efficacy of a medicament administered in combination with the suspension of gas-filled microvesicles. The insonation can be performed at usual conditions. For instance, insonation at a frequency of from 0.1 MHz to 15 MHz, preferably 0.15 - 5 MHz, more preferably 0.2 - 2.5 MHz can be used. The acoustic pressure may be from 50 kPa to 10 MPa, preferably from 100 kPa to 5 MPa, more preferably from 100 kPa to 3.5 MPa. The pulse length is adapted to the specific treatment and organ to be treated and can vary e.g. from 1 µs to 120 seconds, preferably up to 60 seconds, more preferably up to one second.

The following examples will help to further illustrate the invention.

### EXAMPLES

### Materials

| | |
|---|---|
| Definity^{®} | Lantheus Medical Imaging Inc |
| SonoVue^{®} | Bracco Imaging SpA |
| CY^{©}5.5 Mono NHS Ester (fluorescent dye) | GE Healthcare |
| DSPC | Distearoylphosphatidylcholine (from: Lipoid) |
| DPPE-MPEG5000 | Dipalmitoylphosphatidylethanolamine-polyethylenglycol (MW=5000 g/mol) (from: Lipoid) |
| Palmitic Acid | From Lipoid |
| PEG4000 | Polyethylene glycol (MW 4000 g/l) (from: Clariant) |

### Example 1

### Preparation of Experimental Formulation (F01)

The procedure illustrated in the working examples of WO2004/069284 was used for preparing an experimental formulation (F01) for use in subsequent experiments.

Briefly, an emulsion of cyclooctane and water (about 1.5/100 v/v) containing about 90 mg/l of DSPC, 7 mg/l of palmitic acid, 60 mg/l of DPPE-PEG5000 and 100 g/l of PEG4000 was prepared (Megatron MT3000, Kinematica; 10'000 rpm) and sampled into DIN8R vials (about 1 ml/vial).

The vials were cooled at -50°C under vacuum and then subjected to lyophilization, followed by secondary drying above room temperature until complete removal of water and solvent (less than 0.5% by weight). At the end of the freeze-drying process, the headspace of the vials is saturated with a 35/65 mixture of C₄F₁₀/N₂ and the vials were stoppered and sealed.

Before experiments, vials were reconstituted with 1 ml of saline.

### Example 2

### Therapeutic efficacy of experimental Formulation F01

### General setup

CY^{©}5.5 Mono NHS Ester was previously hydrolyzed using a basic buffer (phosphate 100 mM, saline 150 mM, pH 8) over night at room temperature. Anesthetized animal (Male Sprague Dawley Rat) received an intravenous injection of hydrolyzed Cy^{©}5.5 (Cy5.5h) in order to obtain an initial plasmatic concentration of 3.3 µM. Then, serial bolus injections of gas-filled microvesicles (as specified in the subsequent examples) were performed and the left hindlimb muscle was submitted to therapeutic insonation (frequency, acoustic pressure and pulse characteristics as specified in the subsequent examples).

Boluses were spaced by 5 min and total duration of the procedure ranged from 10 to 20 min.

In the following examples 2a and 2b, the ability to induce Cy5.5h extravasation of the gas filled microvesicles of the invention (F01, comprising a mixture of DSPC, DPPE-MPEG5000 and palmitic acid ) was compared under the same conditions with Sonovue^{®} (DSPC, DPPG, Palmitic Acid mixture) and Definity^{®} (DPPC, DPPA, DPPE-MPEG5000 mixture), under different ultrasound conditions (frequency 0.5 MHz to 1.6 MHz; acoustic pressures 170 kPa to 800 kPa).

The therapeutic effect of the combination the gas-filled microvesicles and ultrasound was evaluated by measuring the level of extravasation of Cy5.5h in hindlimb muscles, 120 min after its injection. This was achieved by ex vivo quantitative measurement of Cy5.5h fluorescence signal by near-infrared fluorescence imaging (Fluobeam^{®}700 Fluoptics Grenoble) on fresh tissue. The efficacy of the treatment was considered positive when the mean fluorescence signal ratio between the treated muscles and the control muscles (right hindlimb, not subjected to insonation) was higher than 1.5.

### Example 2a

Each animal (see above "General setup" section) received four subsequent bolus injections of F01, Sonovue or Definity, at a concentration of about 6x10⁷ microvesicles per kg of animal for each bolus, at intervals of five minutes to each other.

Five animals were tested for each formulation.

Left hindlimb muscles were subjected to ultrasound sonication at 800 kPa, 1.6 MHz, 1 ms ON - 4 sec OFF for twenty minutes.

As observable from the results in figure 1 (column c), the fluorescence signal in the muscles treated with ultrasound in combination with F01 was at least twice with respect to the signal measured in the control muscles.. To the contrary, SonoVue or Definity gas-filled microvesicles were not able to induce Cy5.5h extravasation when injected each 5 minutes in the same conditions (columns a and b, respectively).

### Example 2b

The same experiment of example 2a was repeated, with F01 and Definity, by administering two subsequent boluses (at five minutes intervals), each at a concentration of about 1x10⁹ microvesicles per kg of animal.

Three and four animals were tested for F01 and Definity, respectively.

Left hindlimb muscles were subjected to ultrasound sonication at 170 KPa and 0.5 MHz (1 ms ON - 4 sec OFF) for ten minutes.

Results in figure 2 show that the fluorescence signal in the muscles treated with F01 and ultrasound (column b) was more than twice with respect to the signal measured in the control muscles. To the contrary, Definity gas-filled microvesicles were not able to induce Cy5.5h extravasation when injected each 5 minutes in the same conditions (columns a).

The above experimental results show the higher efficacy of the composition of the invention when administered as subsequent boluses (with respect to commercial microvesicles compositions) for increasing the vessel permeabilization in combination with ultrasound treatment, at different conditions of ultrasound insonation.

### List of cited References

**Ref. 1** : Wang, Y. et al. (2018). "Clinical study of ultrasound and microbubbles for enhancing chemotherapeutic sensitivity of malignant tumors in digestive system." Chin J Cancer Res 30(5): 553-563
**Ref. 2:**Auboire, L. et al. (2018). "Microbubbles combined with ultrasound therapy in ischemic stroke: A systematic review of in-vivo preclinical studies." PLoS One 13(2): e0191788.
**Ref. 3:** Huang, Y. et al. (2017). "Opening the Blood-Brain Barrier with MR Imaging-guided Focused Ultrasound: Preclinical Testing on a Trans-Human Skull Porcine Model." Radiology: Volume 282: Number 1, 123-130.
**Ref. 4:** Tu, J. et al. (2018). "Ultrasound-mediated microbubble destruction: a new method in cancer immunotherapy." OncoTargets and Therapy Vol. 11: 5763-5775.
**Ref. 5:** Blackmore, J. et al. (2019). "Ultrasound Neuromodulation: A Review of Results, Mechanisms and Safety." Ultrasound Med Biol 45(7): 1509-1536.
**Ref. 6:** Deng, H. et al. (2018). "Ultrasound-Stimulated Microbubbles Enhance Radiosensitization of Nasopharyngeal Carcinoma." Cell Physiol Biochem 48(4): 1530-1542.
**Ref. 7:** Arvanitis c et al. (2016). "Cavitation-enhanced nonthermal ablation in deep brain targets: feasibility in a large animal model". J Neurosurg.; 124(5): 1450-1459.
**Ref. 8:** Thomas, E. et al. (2019). "Ultrasound-mediated cavitation enhances the delivery ofan EGFR-targeting liposomal formulation designed for chemo-radionuclide therapy". Theranostics 2019, Vol. 9, Issue 19, 5595-5609
**Ref. 9:** Dimcevski, G. et al. (2011). "A human clinical trial using ultrasound and microbubbles to enhance gemcitabine treatment of inoperable pancreatic cancer". J. Controlled Release 243 (2016) 172-181
**Ref. 10:** Schneider, M. et al. (2011). "BR38, a New Ultrasound Blood Pool Agent". Inv. Radiology • Vol. 46, Number 8, 486-494

## Claims

1. A suspension of gas-filled microvesicles comprising a fatty acid di-ester of phosphatidylcholine (PC), a pegylated phosphatidylethanolamine (PE-PEG) and a fatty acid for use in a therapeutic method, wherein said suspension is administered as at least two subsequent boluses at an interval of at least four minutes and up to fifteen minutes between each other.

2. The suspension for use according to claim 1 wherein said at least two subsequent boluses are administered at an interval of at least five minutes between each other.

3. The suspension for use according to claim 1 or 2 wherein said at least two subsequent boluses are administered at an interval of up to ten minutes between each other

4. The suspension for use according to any of the preceding claims wherein the relative molar ratio PC/PE-PEG/Palmitic acid is of 60-90/2-10/5-30.

5. The suspension for use according to claim 4, wherein said ratio is 70-80/3-9/10-25.

6. The suspension for use according to any of the preceding claims wherein said gas-filled microvesicles comprise DSPC, DPPE-MPEG-5000 and palmitic acid.

7. The suspension for use according to any of the preceding claims wherein said gas-filled microvesicles comprise a fluorinated gas.

8. The suspension for use according to any of the preceding claims wherein said method comprises applying ultrasound.

9. The suspension for use according to claim 8 wherein said method comprises increasing vessels permeabilization in a subject undergoing said therapeutic method.

10. The suspension for use according to any of the preceding claims wherein the total amount of microvesicles for each injected volume of suspension is from 6x10⁵ to 2x10⁹ microvesicles per kg of a subject undergoing said therapeutic method.

11. The suspension for use according to any of the preceding claims wherein the concentrations of microvesicles in the injected suspension is of from 1x10⁸ to 2x10⁹ microvesicles/mL

## Patentansprüche

1. Suspension von gasgefüllten Mikrovesikeln, die einen Fettsäurediester von Phosphatidylcholin (PC), ein pegyliertes Phosphatidylethanolamin (PE-PEG) und eine Fettsäure umfassen, zur Verwendung in einem therapeutischen Verfahren, wobei die Suspension als mindestens zwei aufeinanderfolgende Boli in einem Intervall von mindestens vier Minuten und bis zu fünfzehn Minuten zwischen einander verabreicht wird.

2. Suspension zur Verwendung nach Anspruch 1, wobei die mindestens zwei aufeinanderfolgenden Boli in einem Intervall von mindestens fünf Minuten zwischen einander verabreicht werden.

3. Suspension zur Verwendung nach Anspruch 1 oder 2, wobei die mindestens zwei aufeinanderfolgenden Boli in einem Intervall von bis zu zehn Minuten zwischen einander verabreicht werden.

4. Suspension zur Verwendung nach einem der vorhergehenden Ansprüche, wobei das relative Molverhältnis PC/PE-PEG/Palmitinsäure 60-90/2-10/5-30 beträgt.

5. Suspension zur Verwendung nach Anspruch 4, wobei das Verhältnis 70-80/3-9/10-25 beträgt.

6. Suspension zur Verwendung nach einem der vorhergehenden Ansprüche, wobei die gasgefüllten Mikrovesikel DSPC, DPPE-MPEG-5000 und Palmitinsäure umfassen.

7. Suspension zur Verwendung nach einem der vorhergehenden Ansprüche, wobei die gasgefüllten Mikrovesikel ein fluoriertes Gas umfassen.

8. Suspension zur Verwendung nach einem der vorhergehenden Ansprüche, wobei das Verfahren die Anwendung von Ultraschall umfasst.

9. Suspension zur Verwendung nach Anspruch 8, wobei das Verfahren das Erhöhen der Gefäßpermeabilisierung in einem sich dem therapeutischen Verfahren unterziehenden Subjekt umfasst.

10. Suspension zur Verwendung nach einem der vorhergehenden Ansprüche, wobei die Gesamtmenge an Mikrovesikeln für jedes injizierte Suspensionsvolumen 6x10⁵ bis 2x10⁹ Mikrovesikel pro kg eines sich dem therapeutischen Verfahren unterziehenden Subjekts beträgt.

11. Suspension zur Verwendung nach einem der vorhergehenden Ansprüche, wobei die Konzentrationen an Mikrovesikeln in der injizierten Suspension von 1x10⁸ bis 2x10⁹ Mikrovesikel/ml betragen.

## Revendications

1. Suspension de microvésicules remplies de gaz comprenant un diester d'acide gras de phosphatidylcholine (PC), une phosphatidyléthanolamine pégylée (PE-PEG) et un acide gras pour utilisation dans un procédé thérapeutique, dans laquelle ladite suspension est administrée sous forme d'au moins deux bolus subséquents à un intervalle d'au moins quatre minutes et jusqu'à quinze minutes l'un de l'autre.

2. Suspension pour utilisation selon la revendication 1, dans laquelle lesdits au moins deux bolus subséquents sont administrés à un intervalle d'au moins cinq minutes l'un de l'autre.

3. Suspension pour utilisation selon la revendication 1 ou 2, dans laquelle lesdits au moins deux bolus subséquents sont administrés à un intervalle allant jusqu'à dix minutes l'un de l'autre.

4. Suspension pour utilisation selon l'une quelconque des revendications précédentes, dans laquelle le rapport molaire relatif PC/PE-PEG/acide palmitique est de 60-90/2-10/5-30.

5. Suspension pour utilisation selon la revendication 4, dans laquelle ledit rapport est de 70-80/3-9/10-25.

6. Suspension pour utilisation selon l'une quelconque des revendications précédentes, dans laquelle lesdites microvésicules remplies de gaz comprennent DSPC, DPPE-MPEG-5000 et de l'acide palmitique.

7. Suspension pour utilisation selon l'une quelconque des revendications précédentes, dans laquelle lesdites microvésicules remplies de gaz comprennent un gaz fluoré.

8. Suspension pour utilisation selon l'une quelconque des revendications précédentes, dans laquelle ledit procédé comprend l'application d'ultrasons.

9. Suspension pour utilisation selon la revendication 8, dans laquelle ledit procédé comprend l'augmentation de la perméabilisation des vaisseaux chez un sujet subissant ledit procédé thérapeutique.

10. Suspension pour utilisation selon l'une quelconque des revendications précédentes, dans laquelle la quantité totale de microvésicules pour chaque volume injecté de suspension est de 6x10⁵ à 2x10⁹ microvésicules par kg d'un sujet subissant ledit procédé thérapeutique.

11. Suspension pour utilisation selon l'une quelconque des revendications précédentes, dans laquelle les concentrations de microvésicules dans la suspension injectée sont de 1x10⁸ à 2x10⁹ microvésicules/mL.
